# EUROPEAN PATENT APPLICATION

(11) **EP 4 467 485 A1**
(43) Date of publication of application: **27.11.2024**
(21) Application number: 23742737.2
(22) Date of filing: 09.01.2023
(51) Int. Cl.: B65D 83/76, B05B 11/00, A61M 15/00

(54) **TRIGGER ASSEMBLY FOR ATOMIZER, AND ATOMIZER**

(30) Priority: 21.01.2022 CN 202210074351
(71) Applicant: Atsenbo (Suzhou) Pharmaceutical Technology Co., Ltd, Suzhou, Jiangsu 215151 (CN)
(72) Inventor: REN, Hongxian, Suzhou, Jiangsu 215151 (CN)
(74) Representative: Schenk, Markus
(86) International application number: PCT/CN2023/071194
(87) International publication number: WO 2023/138422

(57) **Abstract**

Provided are a trigger assembly for an atomizer, and an atomizer. The trigger assembly comprises a trigger ring (1000) and a delivery tube holder (2000). The trigger ring (1000) comprises a ring body (1200) and a first locking portion arranged on the ring body (1200), and at least one surface of the first locking portion forms a first locking surface (1110). The delivery tube holder (2000) comprises a tube holder body (2200) and a second locking portion arranged on the tube holder body (2200), and at least one surface of the second locking portion forms a second locking surface (2110). The trigger assembly has a locked position and an unlocked position. The trigger assembly can prevent undesired automatic triggering of the atomizer, improving the stability of the atomizer.

## Description

### Technical Field

The present disclosure relates to a trigger assembly for an atomizer, and an atomizer. The atomizer may be used, for example, for atomizing and/or spraying a liquid medicine.

### Background Art

In the related art, a container of an atomizer (or referred to as a sprayer) contains a liquid to be atomized or sprayed, and during movement of the container relative to a spraying assembly (such as a pump), the liquid in the container is atomized and the atomized liquid is sprayed from a nozzle of the spraying assembly. However, the triggering of the atomizer is usually not stable enough, so that the atomization and spraying process of the liquid is often not smooth enough. In addition, when the atomizer is subjected to vibration or shaking, such as accidental slipping, internal assemblies may automatically move undesirably, causing the atomizer to be automatically triggered without human intervention.

This accidental automatic triggering of the atomizer is undesired, which not only leads to an ineffective waste of the liquid, but may also lead to leakage, or even damage or failure of the atomizer when a user is not using the atomizer.

### Summary of the Invention

An objective of the present disclosure is to provide a trigger assembly for an atomizer. The trigger assembly can control whether the atomizer is triggered by switching between a locked position and an unlocked position, thereby preventing undesired automatic triggering of the atomizer and improving the stability of the atomizer.

According to a first aspect of the present disclosure, a trigger assembly for an atomizer is provided. Herein, the trigger assembly comprises a trigger ring and a delivery tube holder, wherein the trigger ring comprises a ring body and a first locking portion arranged on the ring body, and at least one surface of the first locking portion forms a first locking surface; wherein the delivery tube holder comprises a tube holder body and a second locking portion arranged on the tube holder body, and at least one surface of the second locking portion forms a second locking surface; wherein the trigger assembly has a locked position and an unlocked position; in the locked position, the first locking surface abuts against the second locking surface, so that the trigger ring and the delivery tube holder stop each other; and in the unlocked position, the first locking surface is not in contact with the second locking surface, so that the delivery tube holder can move in an axial direction relative to the trigger ring.

The trigger assembly according to the present disclosure can effectively prevent the atomizer from being accidentally triggered without human intervention. For example, during accidental slipping of the atomizer, the trigger assembly can lock a relatively moving component in the atomizer, so that the atomizer is stably located in a non-triggered position. It is further preferred that these relatively moving components are locked to each other, that is, in the locked position of the trigger assembly, the trigger ring locks the movement of the delivery tube holder, and the delivery tube holder also locks the movement of the trigger ring at the same time. Therefore, the locking of the trigger assembly is efficient and firm, thereby effectively and reliably preventing accidental triggering of the atomizer.

In an implementation, the first locking portion is in the form of a locking protrusion extending out from at least one side of the trigger ring, and the second locking portion is in the form of a locking notch recessed on at least one side of the tube holder body.

In another implementation, the first locking portion is in the form of a locking notch recessed on at least one side of the trigger ring, and the second locking portion is in the form of a locking protrusion extending out from at least one side of the tube holder body.

Preferably, the trigger ring and the delivery tube holder stopping each other consists in that the trigger ring prevents a vertical movement of the delivery tube holder, and that the delivery tube holder prevents a horizontal and/or vertical movement of the trigger ring.

Preferably, in the unlocked position, the delivery tube holder can move through the trigger ring in the axial direction.

Preferably, an outer diameter of the delivery tube holder is less than or equal to an inner diameter of the trigger ring.

Preferably, in the locked position, the trigger ring is arranged non-coaxially with respect to the delivery tube holder, and in the unlocked position, the trigger ring is arranged coaxially with respect to the delivery tube holder.

Preferably, the locking protrusion extends outwardly from a bottom side of the ring body of the trigger ring, and the locking notch is arranged on a top side of the tube holder body of the delivery tube holder.

Preferably, the first locking surface is in the form of a radially outward surface of the locking protrusion, and the second locking surface is in the form of a radially inward surface of the delivery tube holder.

Preferably, only one locking protrusion is provided on at least one side of the trigger ring, and a plurality of locking notches, such as two locking notches, are provided on at least one side of the delivery tube holder.

Preferably, the plurality of locking notches are arranged at equal angular intervals in a circumferential direction on a side of the delivery tube holder that mates with the locking protrusion.

Preferably, at least one of the first locking surface and the second locking surface is in the form of a flat inclined surface, and the inclined surface is inclined with respect to both a horizontal direction and a vertical direction.

Preferably, the first locking surface forms a first inclined locking surface, and the second locking surface forms a second inclined locking surface.

Preferably, the first inclined locking surface is in the form of a substantially trapezoidal inclined surface, and the second locking surface is in the form of a substantially parallelogram-shaped inclined surface.

Preferably, an included angle between a normal direction of the first locking surface and a first horizontal direction of the trigger ring is in a range of 135° to 178°, preferably in a range of 155° to 175°; an included angle between the normal direction of the first locking surface and a second horizontal direction of the trigger ring is in a range of 65° to 90°, preferably in a range of 70° to 85°; and an included angle between the normal direction of the first locking surface and a vertical direction of the trigger ring is in a range of 115° to 145°, preferably in a range of 120° to 140°. Herein, the "first horizontal direction" and the "second horizontal direction" of the trigger ring correspond to radial directions of the trigger ring, the "second horizontal direction" particularly corresponds to a trigger direction of the trigger ring, while the "vertical direction" of the trigger ring corresponds to an axial direction of the trigger ring.

Preferably, an included angle between a normal direction of the second locking surface and a first horizontal direction of the delivery tube holder is in a range of 55° to 90°, preferably in a range of 67° to 87°; an included angle between the normal direction of the second locking surface and a second horizontal direction of the delivery tube holder is in a range of 145° to 185°, preferably in a range of 155° to 178°; and an included angle between the normal direction of the second locking surface and a vertical direction of the delivery tube holder is in a range of 40° to 55°, preferably in a range of 45° to 55°. Herein, the "first horizontal direction" and the "second horizontal direction" of the delivery tube holder correspond to radial directions of the delivery tube holder, while the "vertical direction" of the delivery tube holder corresponds to an axial direction of the delivery tube holder.

Preferably, an included angle between a projection of the first locking surface on a vertical plane and the axial direction of the trigger ring is in a range of 15° to 80°, preferably in a range of 30° to 50°.

Preferably, an included angle between a projection of the second locking surface on a horizontal plane and a first radial direction of the delivery tube holder is in a range of 5° to 60°, preferably in a range of 10° to 30°; and an included angle between a projection of the second locking surface in a horizontal direction and a second radial direction of the delivery tube holder is in a range of 5° to 60°, preferably in a range of 5° to 25°.

Preferably, an included angle between a projection of the second locking surface on a vertical plane and an axial direction (Z) of the delivery tube holder is in a range of 15° to 80°, preferably in a range of 35° to 55°.

Preferably, at least one of the first locking surface and the second locking surface is in the form of a curved surface.

Preferably, the delivery tube holder further comprises an inclined guide surface on a side where the locking notch is provided, and the inclined guide surface has an edge shared with the second locking surface.

Preferably, a radial wall thickness dimension of the second locking surface is greater than a half of a radial dimension of the delivery tube holder, and preferably greater than two thirds of the radial dimension of the delivery tube holder.

Preferably, the inclined guide surface and the second locking surface jointly form a wall of the locking notch.

Preferably, an included angle between a projection of the inclined guide surface in a horizontal direction and a second radial direction of the delivery tube holder is equal to an included angle between a projection of the second locking surface in a horizontal direction and the second radial direction of the delivery tube holder.

Preferably, an included angle between a projection of the inclined guide surface on a horizontal plane and a first radial direction of the delivery tube holder is in a range of 20° to 80°, preferably in a range of 40° to 65°.

According to a second aspect of the present disclosure, an atomizer is provided. Herein, the atomizer comprises a trigger assembly as described above, for triggering the atomizer to spray an atomized liquid. In this way, the atomizer according to the present disclosure is stable, because the trigger assembly can effectively prevent, even if the atomizer is subjected to an abnormal operation such as an impact, vibration or shaking, or even accidental slipping, the atomizer from being automatically triggered during the abnormal operation, thus improving the stability of the atomizer in a non-use state.

Preferably, the trigger ring is in the form of a switch button of the atomizer, or the trigger ring is connected to the switch button of the atomizer, so that the trigger ring can move as the switch button is pressed.

Preferably, the delivery tube holder is in the form of a housing of the atomizer, or the delivery tube holder is connected to the housing of the atomizer, so that the delivery tube holder can rotate as the housing rotates.

Preferably, when the atomizer is in an initial position, the trigger assembly is located in the unlocked position; and when the atomizer is in a pre-trigger position, the trigger assembly is located in the locked position.

Preferably, the delivery tube holder can move vertically in the unlocked position, such that the atomizer moves from the initial position to the pre-trigger position, and the trigger ring can be pressed in the locked position to move horizontally, such that the atomizer moves from the pre-trigger position back to the initial position.

Preferably, the atomizer further comprises a spring at a bottom of the delivery tube holder, for applying a thrust to the delivery tube holder to urge the delivery tube holder to pass through the trigger ring in an axial direction.

Preferably, the atomizer further comprises: a container configured to contain a liquid to be atomized, and a spraying assembly sleeved on the container to suck the liquid from the container and atomize the liquid and spray the atomized liquid.

### Brief Description of the Drawings

In order to more clearly describe the technical solutions in the embodiments of the present disclosure or in the prior art, the accompanying drawings required for describing the embodiments or the prior art are briefly described below. Apparently, the accompanying drawings in the following description merely show some of the embodiments of the present disclosure, and those of ordinary skill in the art may still derive other accompanying drawings according to the structures shown by these accompanying drawings without creative efforts. The figures are as follows:
FIG. 1 is a front view showing a trigger ring according to an implementation of the present disclosure;
FIG. 2 is a top view showing a trigger ring according to an implementation of the present disclosure;
FIG. 3 is a partially enlarged view showing the trigger ring shown in FIG. 2;
FIG. 4 is a perspective view showing a trigger ring according to an implementation of the present disclosure;
FIG. 5 is a front view showing a delivery tube holder according to an implementation of the present disclosure;
FIG. 6 is a top view showing a delivery tube holder according to an implementation of the present disclosure;
FIG. 7 is a partially enlarged view showing the delivery tube holder shown in FIG. 6;
FIG. 8 is a perspective view showing a delivery tube holder according to an implementation of the present disclosure;
FIG. 9 is a front view showing a trigger assembly located in a locked position according to an implementation of the present disclosure;
FIG. 10 is a top view showing a trigger assembly located in a locked position according to an implementation of the present disclosure;
FIG. 11 is a perspective view showing a trigger assembly located in a locked position according to an implementation of the present disclosure;
FIG. 12 is a front view showing a trigger assembly located in an unlocked position according to an implementation of the present disclosure;
FIG. 13 is a top view showing a trigger assembly located in an unlocked position according to an implementation of the present disclosure;
FIG. 14 is a perspective view showing a trigger assembly located in an unlocked position according to an implementation of the present disclosure;
FIG. 15 is a front view showing a trigger assembly located in another unlocked position according to an implementation of the present disclosure;
FIG. 16 is a perspective view showing a trigger assembly located in another unlocked position according to an implementation of the present disclosure;
FIG. 17 is an exploded view showing an atomizer according to an implementation of the present disclosure;
FIG. 18 is a schematic view showing an atomizer according to an implementation of the present disclosure; and
FIG. 19 is a sectional view showing the atomizer of FIG. 18 taken along line C-C.

### Detailed Description of Embodiments

The technical solutions in the embodiments of the present disclosure are clearly and completely described below with reference to the accompanying drawings for the embodiments of the present disclosure. Apparently, the described embodiments are merely some rather than all of the embodiments of the present disclosure. Based on the embodiments of the present disclosure, all other embodiments obtained by those of ordinary skill in the art without creative efforts fall within the scope of protection of the present disclosure.

It should be noted that all directional indications (such as up, down, left, right, front, and back) in the embodiments of the present disclosure are only intended to explain a relative positional relationship, a movement status, and the like between components in a specific posture (as shown in the figures). If the specific posture changes, the directional indications change accordingly.

In the present disclosure, unless otherwise clearly specified and limited, the terms such as "connection" and "fixing" should be understood in a board sense, which, for example, may be a direct connection or an indirect connection implemented by means of an intermediate medium, or may be internal communication between two elements or an interaction relationship between two elements, unless otherwise clearly limited. For those of ordinary skill in the art, specific meanings of the foregoing terms in the present disclosure may be understood based on specific situations.

In the present disclosure, unless otherwise specified, all numbers expressing component parameters, technical effects, etc. used in this description and claims should be understood as being modified by the term "about" or "approximately" in any case. Therefore, unless indicated to the contrary, numerical parameters as set forth in the following description and appended claims are approximations. For those skilled in the art, each numerical parameter may vary depending upon the desired properties and effects sought to be obtained by the present disclosure and should be construed in light of the number of significant digits and conventional rounding methods or in a manner understood by those skilled in the art.

The terms used in the description of the various examples in the present disclosure are for the purpose of describing particular examples only and are not intended to be limiting. Unless the context clearly indicates otherwise, if the number of elements is not specifically limited, there may be one or more elements. Moreover, the term "and/or" used in the present disclosure encompasses any of and all possible combinations of listed items.

In the scope of the present disclosure, an "atomizer" (also referred to as a "sprayer") refers to a device for atomizing a liquid. Generally, the atomizer is configured to atomize a fluid (e.g., a liquid medicine or the like) and spray the atomized fluid to some parts of a user (such as a patient) to be treated. Since the liquid medicine is loaded in the atomizer, the stability of the atomizer is particularly important.

In the related art, the atomizer may be operated by mechanical means, such as a push switch or a rotary switch. However, the locking of the switches in a non-use state of the atomizer may be unreliable. In addition, when no atomization is needed, the atomizer may be subjected to some impacts or shaking, or may even accidentally slipping from a high level. These accidents not only make the liquid medicine shake in the atomizer, but may also cause relative movement of components of the atomizer and therefore undesired atomization of the liquid. It may be understood that this undesired atomization trigger is ineffective, because in this case, the liquid medicine does not reach a part to be treated. Even worse, this undesired atomization trigger may be harmful, because the atomized liquid that is accidentally sprayed may flow everywhere and may accumulate in the atomizer, which may lead to functional damage or failure of the components of the atomizer over time.

In view of this, the present disclosure provides an improved trigger assembly, which is mounted in an atomizer, and preferably can be linked with a push switch and/or a rotary switch of the atomizer, so as to control effective triggering of the atomizer and/or prevent ineffective triggering of the atomizer by switching the trigger assembly between a locked position and an unlocked position.

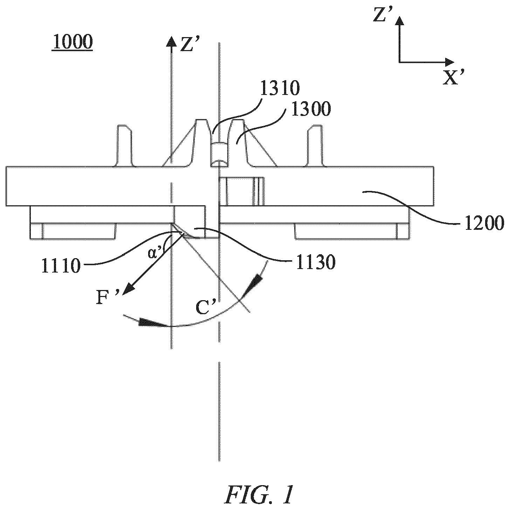

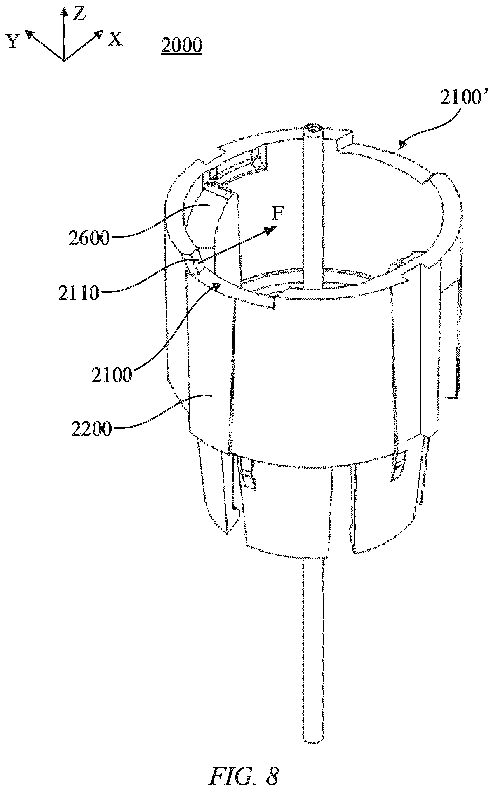

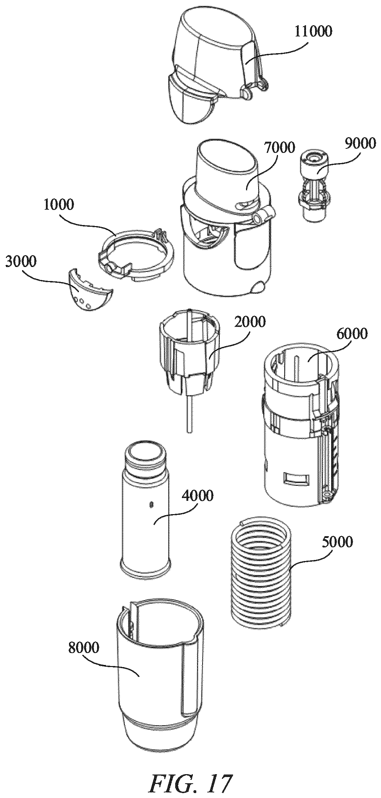

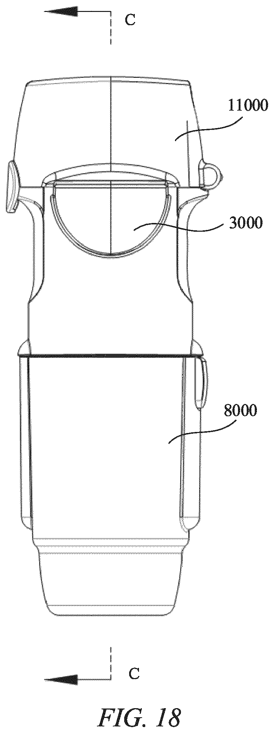

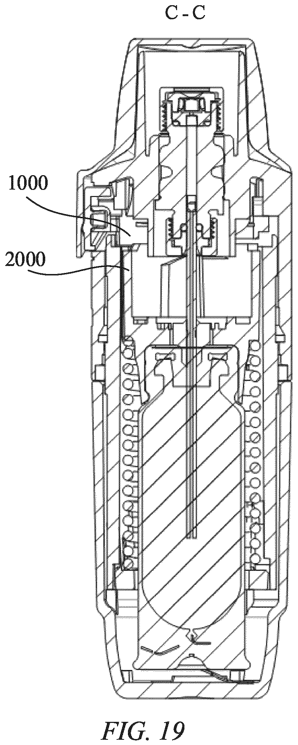

In the scope of the present disclosure, the "trigger assembly" refers to an assembly configured to control triggering of the atomizer, such as an assembly that can control and/prevent an atomization or spraying operation that is performed by the atomizer. In the present disclosure, the trigger assembly may comprise, for example, a trigger ring 1000 and a delivery tube holder 2000, with specific structures described in detail below with reference to FIGS. 1 to 4 and FIGS. 5 to 8, respectively. In addition, the arrangement of the trigger assembly in the atomizer may be described in detail, for example, with reference to FIGS. 17 to 19.

It should be understood that the trigger assembly may comprise not only the trigger ring 1000 and the delivery tube holder 2000, but may also comprise other elements, provided that a function of controlling and/or preventing triggering can be achieved. Importantly, the trigger assembly has (one or more) locked positions and (one or more) unlocked positions, and can be switched therebetween.

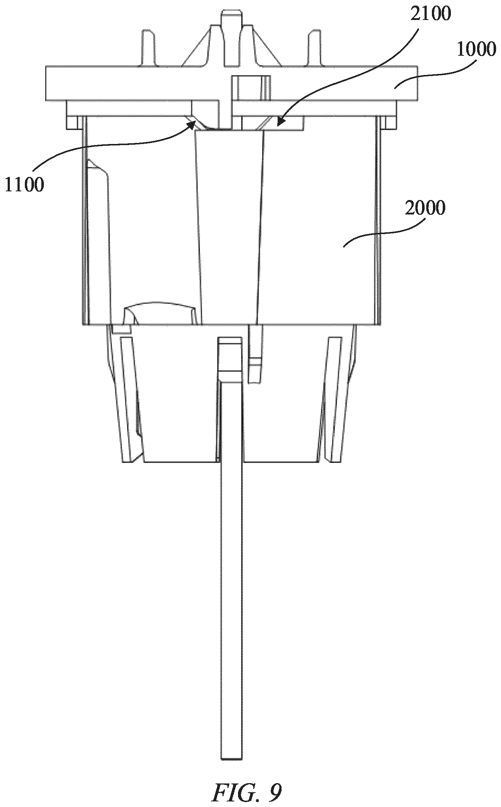

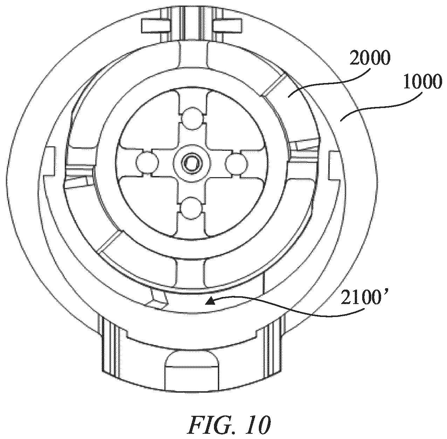

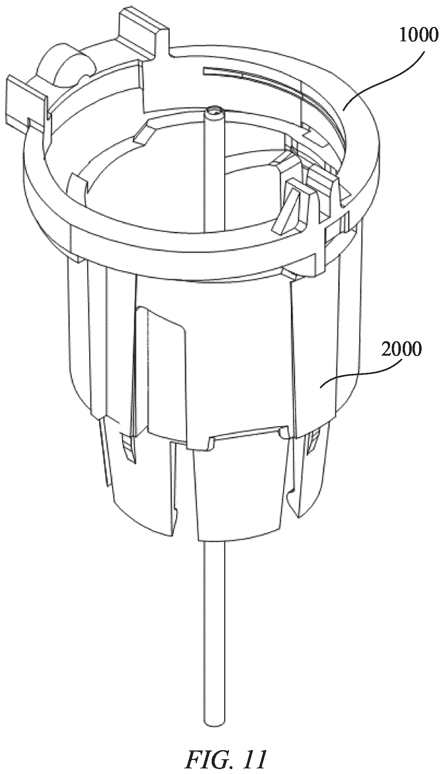

In the scope of the present disclosure, the "locked position" of the trigger assembly refers to a position where the atomizer is locked, that is, a position where the atomizer cannot operate without human intervention. In other words, when the trigger assembly is in a "locked position", the atomizer can perform an atomization or spraying operation only after manual operation. In the present disclosure, the "locked position" of the trigger assembly may alternatively refer to a position where the trigger ring 1000 and the delivery tube holder 2000 stop each other. As an example, by the trigger ring 1000 and the delivery tube holder 2000 "stopping each other" may mean, for example, that the trigger ring 1000 prevents a movement, such as a vertical movement, of the delivery tube holder 2000, while the delivery tube holder 2000 also prevents a further movement, such as a horizontal and/or vertical translation, of the trigger ring 1000. The "locked position" is further described below by virtue of FIGS. 9 to 11.

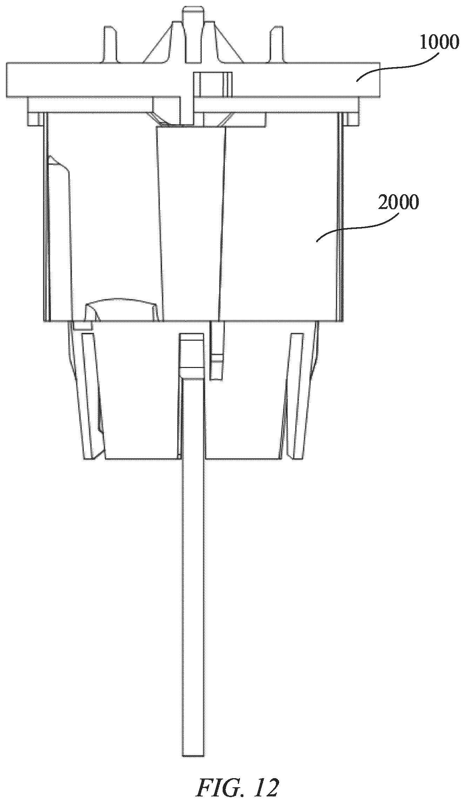

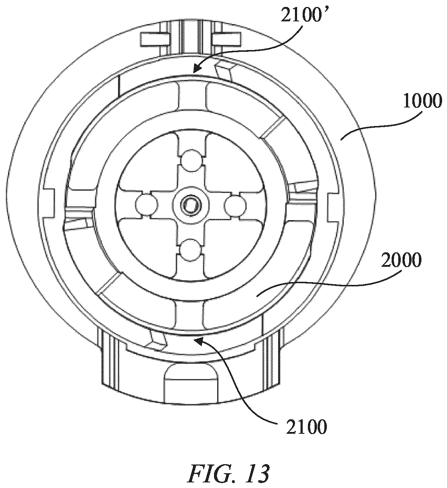

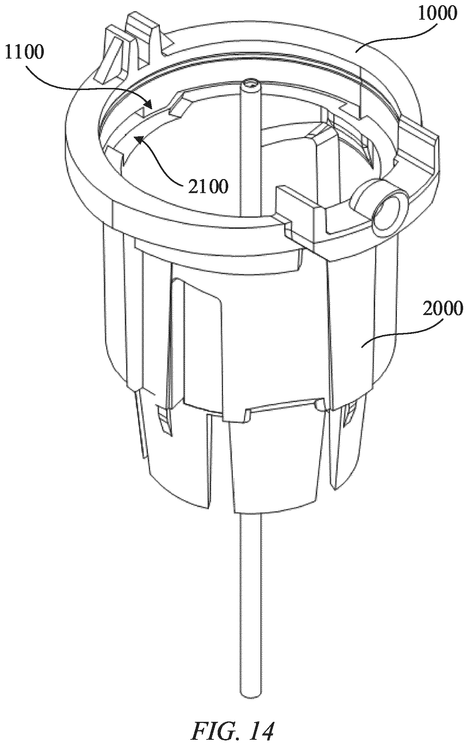

In the scope of the present disclosure, the "unlocked position" of the trigger assembly refers to a position where the atomizer is not locked, that is, an operable position of the atomizer. In other words, when the trigger assembly is in the "unlocked position", components (such as the trigger ring 1000 and the delivery tube holder 2000) in the atomizer can move relative to each other without manual operation, so as to achieve an effect of atomizing the liquid and/or spraying the atomized liquid. It may be understood that the "unlocked position" of the trigger assembly is a concept with respect to the "locked position" described above. As long as the atomizer is not locked, the trigger assembly is located in the unlocked position. Therefore, the trigger assembly may, for example, have a plurality of "unlocked positions", and the trigger assembly may be switched between the locked position and an unlocked position, or between a plurality of unlocked positions. During switching of the trigger assembly, the atomizer can be switched between an initial position, a pre-trigger position, and a triggered position. The "unlocked position" is further described below by virtue of FIGS. 12 to 14 and FIGS. 15 to 16.

First, a trigger ring 1000 according to an implementation of the present disclosure is described with reference to FIGS. 1 to 4. The trigger ring 1000 may comprise a ring body 1200 and a first locking portion (herein, for example, a locking protrusion 1100). The locking protrusion 1100 protrudes downwardly from a bottom side of the ring body 1200. It may be understood that although only one locking protrusion 1100 protruding downwardly from the bottom side of the ring body 1200 is shown in the figures, the number and arrangement of locking protrusions 1100 are not limited thereto. As an example, the trigger ring 1000 may alternatively comprise a plurality of locking protrusions 1100. For example, two or three locking protrusions 1100 are arranged on a side of the trigger ring, and these locking protrusions 1100 are preferably circumferentially arranged at equal angular intervals in an array, so that multi-stage locking and unlocking of the trigger assembly can be achieved, for example. For example, when two locking protrusions 1100 are provided, the two locking protrusions 1100 are arranged at an angular interval of 180° in an array along a circumference of the ring body 1200.

As another example, it is also possible that locking protrusions 1100 are arranged on two sides of the ring body 1200. In this case, the locking protrusions 1100 on an upper side and a lower side are staggered from each other, for example, and respectively mate, and are linked, with locking notches 2100 on the upper side and the lower side of the trigger ring 1000.

It can be further seen in the figures that some rib structures are respectively provided on the two sides of the ring body 1200 for mutual connection or mating with other components. For example, two ribs 1300 on top of the ring body 1200 form a slot 1310. The slot can mate with a rotating member of the atomizer, so as to limit rotation of the ring body 1200 to make the ring body 1200 only translate.

The locking protrusion 1100 may comprise a first locking surface 1110, which is, for example, a lower inclined surface of the locking protrusion 1100. It can be seen that the first locking surface 1110 is an inclined surface that is inclined in three-dimensional directions, that is, the first locking surface 1110 is not only inclined relative to a horizontal plane (an X'Y' plane), but also inclined relative to vertical planes (a Z'Y' plane and an X'Z' plane). In other words, the first locking surface 1110 is both a radially outward surface of the trigger ring 1000 and an axially outward surface of the trigger ring 1000.

As an example, an included angle β' between a normal direction F' of the first locking surface 1110 and a first horizontal direction X' of the trigger ring 1000 is in a range of 135° to 178°, preferably in a range of 155° to 175°, or the included angle β' is in a range of 150° to 189°, especially preferably 168.5°. An included angle γ' between the normal direction F' of the first locking surface 1110 and a second horizontal direction Y' of the trigger ring 1000 is in a range of 65° to 90°, preferably in a range of 65° to 89°, preferably in a range of 70° to 85°, especially preferably 78.5°; and an included angle α' between the normal direction F' of the first locking surface 1110 and a vertical direction Z' of the trigger ring 1000 is in a range of 115° to 145°, preferably in a range of 120° to 140°, especially preferably 130°. In the present disclosure, the "first horizontal direction X'" and the "second horizontal direction Y‴ of the trigger ring 1000 respectively correspond to radial directions of the trigger ring 1000, and the second horizontal direction Y' is also a trigger direction of the trigger ring 1000, that is, a direction in which the trigger ring 1000 moves horizontally due to a thrust when a user presses the push switch. The "vertical direction Z'" of the trigger ring 1000 corresponds to an axial direction of the trigger ring 1000. These directions X', Y', and Z' are marked with reference to coordinate systems in FIGS. 1 to 3.

Herein, this three-dimensional inclined arrangement of the first locking surface 1110 is very advantageous. On one hand, when the first locking surface mates with a second locking surface 2110 provided on the locking notch 2100, an operating force for switching the trigger assembly from a locked position to an unlocked position will not be excessively large, otherwise the trigger assembly may be stuck in the unlocked position or the user will need a very great force to remove a mutual stop effect between the trigger ring 1000 and the delivery tube holder 2000. On the other hand, when it is necessary to lock the trigger assembly to, for example, keep the atomizer in a pre-trigger state, the three-dimensionally inclined first locking surface 1110 can effectively abut against the second locking surface 2110 in multiple dimensions to prevent a further vertically upward movement and/or rotational movement thereof. It can be seen that the three-dimensionally inclined first locking surface 1110 achieves a desired balance effect between prevention of being stuck and prevention of automatic atomization.

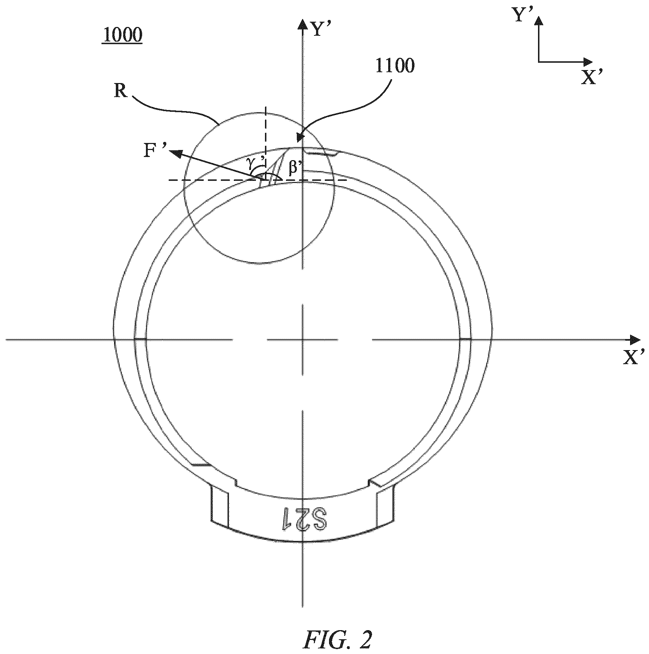

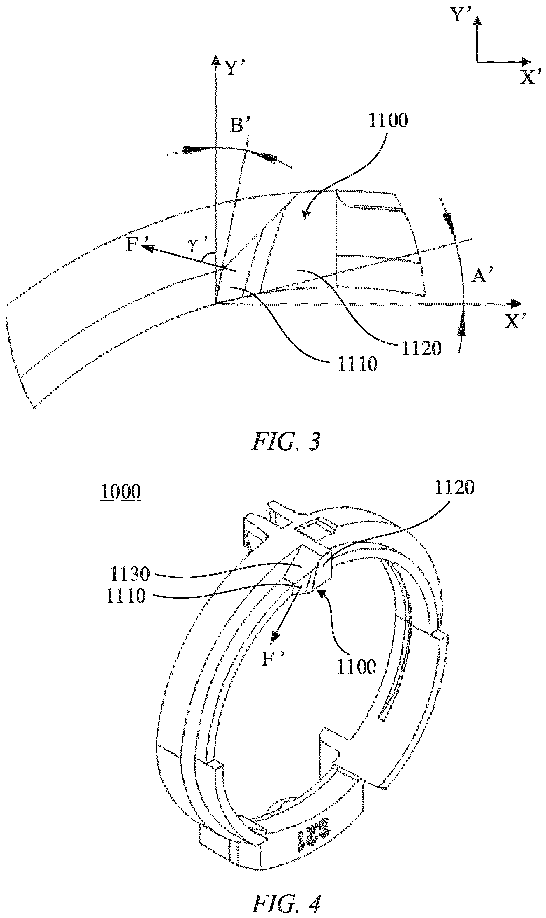

In a further example, FIG. 3 shows a schematic enlarged view of part R of FIG. 2. A projection of the first locking surface 1110, which is generally trapezoidal herein, on a horizontal plane can be seen herein. Herein, an included angle A' between the projection of the first locking surface 1110 and a first radial direction X' of the trigger ring 1000 is in a range of 5° to 50°, preferably in a range of 5° to 30°, for example, about 15°, and an included angle B' between the projection and a second radial direction Y' of the trigger ring 1000 is in a range of 3° to 60°, preferably in a range of 5° to 20°, for example, about 20°. It is particularly preferred when the included angle A' is greater than the included angle B' (for example, the included angle A' is 5° to 10° greater than the included angle B'), so that relative sliding between the locking protrusion 1100 and the locking notch 2100 is smooth and firm during movement of an unlocking assembly from the locked position to the unlocked position. It is also preferred that an included angle C' between a projection of the first locking surface 1110 on a vertical plane and the axial direction Z' of the trigger ring 1000 is in a range of 15° to 80°, preferably in a range of 30° to 50°.

The locking protrusion 1100 further comprises a guide surface 1120, which is herein a bottom surface of the locking protrusion 1100 and is horizontally arranged. Of course, the guide surface 1120 may be alternatively arranged slightly inclined with respect to the horizontal plane. During position switching of the trigger assembly, the guide surface 1120 may be in contact with a groove bottom of the locking notch 2100, thus making the switching process smoother.

In addition, the locking protrusion 1100 further comprises a transition surface 1130 configured to limit rotational movement of the trigger ring 1000, so that the trigger ring 1000 can only translate under the constraint of the transition surface.

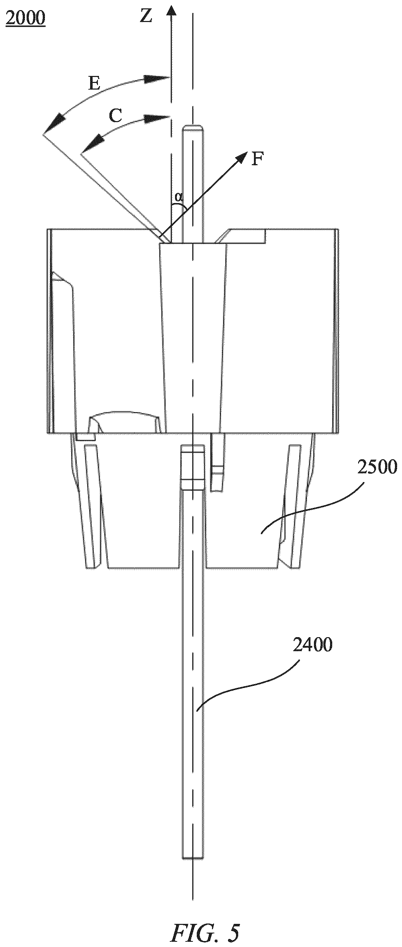

A delivery tube holder 2000 according to an implementation of the present disclosure is described in detail below with reference to FIGS. 5 to 8. The delivery tube holder 2000 may comprise a barrel-shaped tube holder body 2200 and a second locking portion (herein, for example, a locking notch 2100 recessed on a side of the tube holder body 2200) arranged on the tube holder body. Although two locking notches 2100 and 2100' are shown in the figures, it should be understood that the number and arrangement of locking notches 2100 are not restrictive, but any number of locking notches 2100 may be provided on an upper wall of the tube holder body 2200, and a different number of second locking surfaces 2110 may be provided on a wall of one or either side of each of the locking notches 2100. The possibility of flexible arrangement of the locking notches 2100 enables a motion level of the unlocking assembly to be adapted as required.

Although in this embodiment, the locking protrusion 1100 is used as an example of the first locking portion and the locking notch 2100 is used as an example of the second locking portion, it is also conceivable that in another embodiment, the first locking portion is in the form of a locking notch, while the second locking portion is in the form of a locking protrusion. It should be understood that as long as the first locking portion and the second locking portion can achieve interlocking and unlocking functions, the arrangements of the locking protrusion and the locking notch are interchangeable or can be selectively used as required.

At least one wall of the locking notch 2100 forms a second locking surface 2110, and the second locking surface 2110 mates with the first locking surface 1110 described above. That is, when the trigger assembly is located in the locked position, the second locking surface 2110 and the first locking surface 1110 abut against each other. As the trigger ring 1000 moves, for example, under the action of the push switch, the first locking surface 1110 abuts against the second locking surface 2110 and slides thereon, and when the first locking surface slides past the second locking surface 2110, the trigger assembly enters the unlocked position.

Similar to the first locking surface 1110, the second locking surface 2110 is also a three-dimensionally inclined surface, that is, the second locking surface 2110 is not only inclined relative to a horizontal plane (an XY plane), but also inclined relative to vertical planes (a ZY plane and an XZ plane). In other words, the second locking surface 2110 is both a radially inward surface of the delivery tube holder 2000 and an axially inward surface of the delivery tube holder 2000.

As an example, an included angle β between a normal direction F of the second locking surface 2110 and a first horizontal direction X of the delivery tube holder 2000 is in a range of 55° to 90°, preferably in a range of 67° to 87°, or preferably in a range of 60° to 90°, especially preferably 77°; an included angle γ between the normal direction F of the second locking surface 2110 and a second horizontal direction Y of the delivery tube holder 2000 is in a range of 145° to 185°, preferably in a range of 150° to 180°, preferably in a range of 155° to 178°, especially preferably 167°; and an included angle α between the normal direction F of the second locking surface 2110 and a vertical direction Z of the delivery tube holder 2000 is in a range of 30° to 60°, preferably in a range of 40° to 55°, especially preferably 46.3°. In the present disclosure, the "first horizontal direction X" and the "second horizontal direction Y" of the delivery tube holder 2000 correspond to radial directions of the delivery tube holder 2000, while the "vertical direction Z" of the delivery tube holder 2000 corresponds to an axial direction of the delivery tube holder 2000. These directions X, Y, and Z are marked with reference to coordinate systems in FIGS. 5 to 8.

Herein, this three-dimensional inclined arrangement of the second locking surface 2110 is very advantageous. On one hand, when the second locking surface mates with the first locking surface 1110, an operating force for switching the trigger assembly from a locked position to an unlocked position will not be excessively large, otherwise the trigger assembly may be stuck in the unlocked position or the user will need a very great force to remove a mutual stopping effect between the trigger ring 1000 and the delivery tube holder 2000. On the other hand, when it is necessary to lock the trigger assembly to, for example, keep the atomizer in the pre-trigger state, the three-dimensionally inclined second locking surface 2110 can effectively abut against the first locking surface 1110 in multiple dimensions to prevent a further movement thereof. It can be seen that the three-dimensionally inclined second locking surface 2110 achieves a desired balance effect between prevention of being stuck and prevention of automatic atomization.

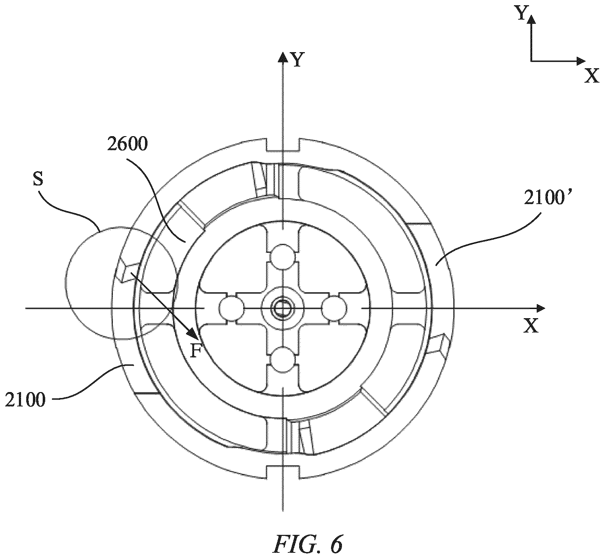

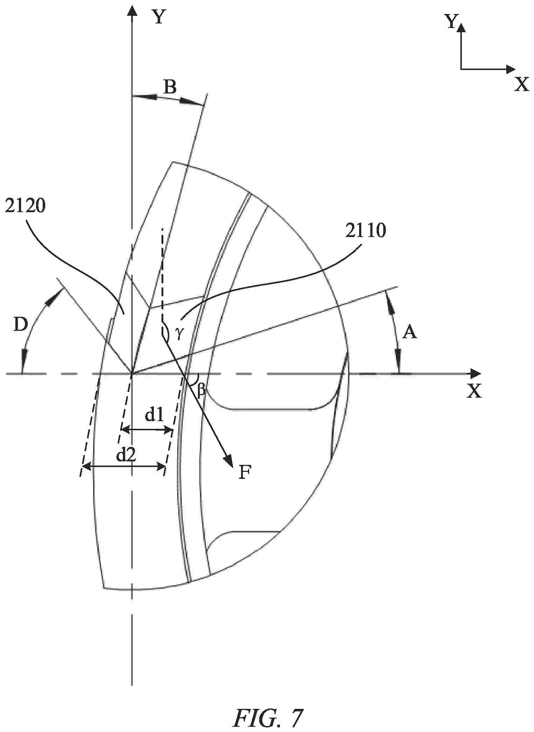

In a further example, FIG. 7 shows a schematic enlarged view of part S of FIG. 6. A projection of the second locking surface 2110, which is generally parallelogram-shaped herein, on a horizontal plane can be seen herein. Herein, an included angle A between a projection of the second locking surface 2110 on a horizontal plane and a first radial direction X of the delivery tube holder 2000 is in a range of 5° to 60°, preferably in a range of 10° to 45°, for example, 30°; and an included angle B between a projection of the second locking surface 2110 in a horizontal direction and a second radial direction Y of the delivery tube holder 2000 is in a range of 5° to 60°, preferably in a range of 5° to 25°, for example, 15°. It is particularly preferred when the included angle A is greater than the included angle B (for example, the included angle A is 5° to 10° greater than the included angle B), so that a stroke of the unlocking assembly from the locked position to the unlocked position can be controlled. In addition, an included angle C between a projection of the second locking surface 2110 on a vertical plane and an axial direction Z of the delivery tube holder 2000 is in a range of 15° to 80°, preferably in a range of 35° to 55°.

The locking notch 2100 further comprises a groove bottom that may be in contact with the guide surface 1120, and two side walls. The second locking surface 2110 is provided in one wall. As can also be seen from the figure, in a preferred embodiment, the locking notch 2100 further comprises an inclined guide surface 2120, the inclined guide surface 2120 has an edge shared with the second locking surface 2110, and a sum of a radial dimension d1 of the second locking surface 2110 and a radial dimension of the inclined guide surface 2120 is equal to a radial wall thickness dimension d2 of the tube holder body 2200 on this side. The inclined guide surface 2120 also forms a substantially parallelogram shape and is substantially arranged at the same position with the same angle in the circumferential direction as the second locking surface 2110. Herein, the inclined guide surface 2120 may be configured, for example, to guide the unlocking assembly into the unlocked position, so as to prevent the unlocking ring from deviating too far due to too free movement when the unlocking assembly suddenly leaves the locked position. In the implementation in which the inclined guide surface 2120 is provided, the radial dimension d1 of the second locking surface 2110 is greater than a half of the radial wall thickness dimension d2 of the delivery tube holder 2000 on this side, and preferably greater than two thirds of the radial wall thickness dimension d2 of the delivery tube holder 2000 on this side. A projection of the inclined guide surface 2120 in a horizontal direction is shown in FIG. 7. An included angle between the horizontal projection and a second radial direction Y of the delivery tube holder 2000 is equal to the included angle B between the projection of the second locking surface 2110 in the horizontal direction and the second radial direction Y of the delivery tube holder 2000. Preferably, an included angle D between a projection of the inclined guide surface 2120 on a horizontal plane and the first radial direction X of the delivery tube holder 2000 is in a range of 20° to 80°, preferably in a range of 40° to 65°, for example, 45°. Preferably, an included angle between a normal direction of the inclined guide surface 2120 and a first horizontal direction X of the delivery tube holder 2000 is in a range of 135° to 160°; an included angle between the normal direction of the inclined guide surface 2120 and a second horizontal direction Y of the delivery tube holder 2000 is in a range of 115° to 135°; and an included angle between the normal direction of the inclined guide surface 2120 and a vertical direction Z of the delivery tube holder 2000 is in a range of 115° to 135°.

It may be understood that the inclined guide surface 2120 is used as only a preferred arrangement structure, but is not necessary. When no inclined guide surface 2120 is provided, the second locking surface 2110 may account for the entire radial dimension of the wall thickness.

The delivery tube holder 2000 further comprises a connecting piece 2500 that is connected to other members of the sprayer, such as a container 4000, a rotating member 6000, or a housing 8000, for example, by snap-fit connection. The delivery tube holder 2000 further comprises a delivery tube 2400 configured to deliver a liquid stored in the container 4000 to a spray chamber for spraying.

In a preferred implementation, an elastic member, such as a spring 5000, may be further arranged at a bottom of the delivery tube holder 2000. The spring 5000 always applies an upward thrust to the delivery tube holder 2000, so that once the trigger assembly leaves the locked position, the delivery tube holder 2000 can move upwardly, that is, move axially. When an outer diameter of the delivery tube holder 2000 is less than or equal to an inner diameter of the trigger ring 1000, the delivery tube holder 2000 can pass through the trigger ring 1000 upwardly under the action of the spring 5000, and when a downward force is applied to the delivery tube holder 2000, the delivery tube holder 2000 can move downwardly against the elastic force and return to the locked position. It should be understood that the spring 5000 is a preferred component of the atomizer, but is not necessary. Other alternative components, such as buttons, are also conceivable provided that these components can also provide an axial upward thrust for the delivery tube holder 2000.

The delivery tube holder 2000 may be further internally provided with a spiral portion 2600, which is arranged on an upper surface of a protruding portion radially inward from an inner wall of the tube holder body 2200. When the delivery tube holder 2000 is arranged in the atomizer, the spiral portion 2600 may, for example, mate with a corresponding spiral portion 7100 of a mouthpiece 7000. Therefore, when the delivery tube holder 2000 is rotated, the delivery tube holder 2000 can move in the vertical direction, that is, in the axial direction. In the present disclosure, during downward movement of the delivery tube holder 2000, the delivery tube holder 2000 performs downward rotary movement under the action of the spiral portion, that is, performs a composite motion of rotation and downward translation, while during upward movement of the delivery tube holder 2000, the delivery tube holder 2000 can perform a simple upward translation movement without any rotation.

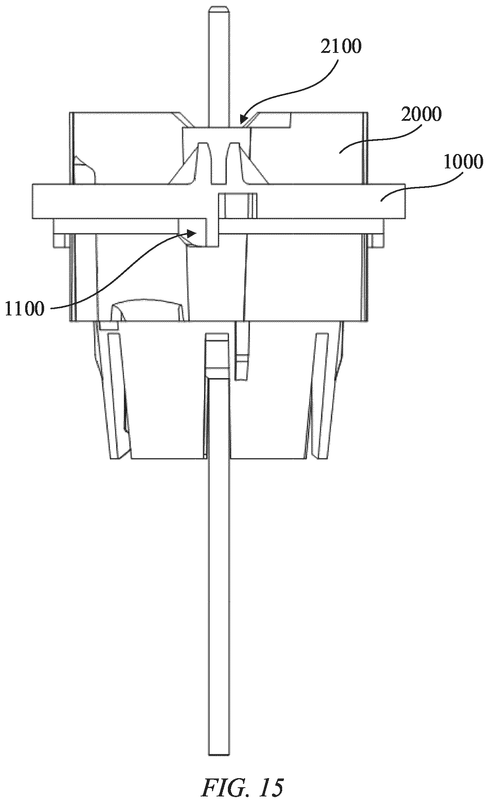

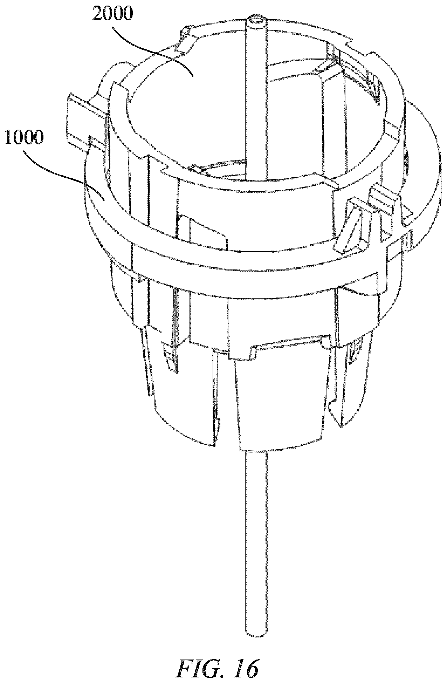

In the present disclosure, the trigger assembly may be composed of, for example, the trigger ring 1000 and the delivery tube holder 2000 as described above, and can move between a locked position and an unlocked position in order to prevent automatic triggering of the atomizer. A locked position of a trigger assembly according to an implementation of the present disclosure is described in detail below with reference to FIGS. 9 to 11, and two unlocked positions of the trigger assembly according to an implementation of the present disclosure are described in detail with reference to FIGS. 12 to 16. FIGS. 12 to 14 show a first unlocked position of the trigger assembly, and FIGS. 15 and 16 show a second unlocked position of the trigger assembly.

The trigger assembly in the locked position is shown in FIGS. 9 to 11. Herein, the locked position, for example, corresponds to a pre-trigger position of the atomizer, that is, when the housing 8000 of the atomizer is rotated, the delivery tube holder 2000 moves downwardly along a spiral surface (preferably moves downwardly while rotating) to a position where the locking protrusion 1100 and the locking notch 2100 stop each other. In this pre-trigger position, the atomizer is stable, that is, the atomizer is prevented from being triggered accidentally, because the locking protrusion 1100 and the locking notch 2100 stop each other, so that any one thereof cannot move without manual operation.

In the locked position, the first locking surface 1110 of the locking protrusion 1100 abuts against the second locking surface 2110 of the locking notch 2100, so as to implement mutual stop of the trigger ring 1000 and the delivery tube holder 2000. As mentioned above, since the first locking surface 1110 and the second locking surface 2110 are both three-dimensionally inclined surfaces, the mutual stop is very reliable and can ensure that unlocking caused by an accidental operation (such as impact on or accidental slipping of the atomizer) does not occur.

In a preferred implementation, in the locked position, the trigger ring 1000 is arranged non-coaxially with respect to the delivery tube holder 2000, that is, the trigger ring and the delivery tube holder are arranged eccentrically to each other.

In order to switch the trigger assembly to the unlocked position, a horizontal thrust is applied to the trigger ring 1000. The horizontal thrust may be indirectly applied to the trigger ring 1000 by manually pressing the push switch, or may be directly applied to the trigger ring 1000 manually.

It should be understood that once the trigger assembly leaves the locked position, the trigger assembly enters the unlocked position. In other words, as long as the atomizer does not need to be locked to prevent accidental triggering, the trigger assembly can reach the unlocked position to facilitate a free operation of the atomizer.

FIGS. 12 to 14 and FIGS. 15 and 16 respectively show the trigger assembly in a first unlocked position and in a second unlocked position. Herein, the "first unlocked position" and the "second unlocked position" are only distinguished for clear explanation, and do not represent a sequential relationship therebetween.

The first unlocked position shown in FIGS. 12 to 14 may be a transition state or a stable state, because when the first locking surface 1110 is no longer in contact with the second locking surface 2110, the delivery tube holder 2000 can move in an axial direction relative to the trigger ring 1000. As mentioned above, since the trigger ring 1000 is pushed horizontally to make the trigger assembly leave the locked position, in the first unlocked position, the trigger ring 1000 is coaxially arranged with respect to the delivery tube holder 2000, that is, there is no eccentricity therebetween. In this case, if the outer diameter of the delivery tube holder 2000 is less than or equal to the inner diameter of the trigger ring 1000, the delivery tube holder 2000 can pass through the trigger ring 1000 to reach the second unlocked position.

In order to urge the delivery tube holder 2000 to pass through the trigger ring 1000, a thrust mechanism, such as a spring 5000, may be arranged below the delivery tube holder 2000. When the spring 5000 is provided, the first unlocked position is a transition state, because once the trigger ring 1000 is pushed horizontally to make the first locking surface 1110 slide past the second locking surface 2110, the delivery tube holder 2000 moves upwardly under the action of the spring 5000. When no spring is provided, the first unlocked position is in a relatively stable state, because even if the trigger ring 1000 is pushed horizontally to make the first locking surface 1110 slide past the second locking surface 2110, the delivery tube holder 2000 is not forced immediately to move upwardly. In this case, other passive operation structures such as a button may be considered.

No matter whether it is due to active pressing by the spring or passive pushing by other alternative structures such as a button, after the delivery tube holder 2000 passes through the trigger ring 1000, the trigger assembly can reach the second unlocked position shown in FIGS. 15 and 16. It may be understood that during switching of the trigger assembly from the first locked position to the second locked position, the delivery tube holder 2000 can perform a simple vertical upward movement, that is, an upward movement without rotation, so as to reach the second unlocked position relative to the trigger ring 1000.

The second unlocked position shown in FIG. 15 may correspond to an initial state of the atomizer, that is, a state in which no operation is performed on the atomizer. When the delivery tube holder 2000 rotates with the housing 8000 and the rotating member 6000 from the initial state, the delivery tube holder 2000 pivots downwardly with respect to the trigger ring 1000 under the action of the spiral surface 2600 until the first locking surface 1110 abuts against the second locking surface 2110, reaching the locked position shown in FIG. 9, that is, the pre-trigger state of the atomizer. In this case, by application of a substantially horizontal thrust to the trigger ring 1000, the trigger assembly can leave the locked position to reach the first unlocked position shown in FIG. 12, thereby completing a stroke cycle.

It may be understood that the stroke cycle of the trigger assembly may correspond to a full-circle rotation or a part-circle rotation of the trigger ring 1000 and the delivery tube holder 2000, depending on the corresponding number and arrangement positions of locking protrusions 1100 and locking notches 2100 provided on the trigger ring 1000 and the delivery tube holder 2000.

Finally, an atomizer according to an example of the present disclosure is described with reference to FIGS. 17 to 19. In order to control or prevent triggering of the atomizer, the atomizer comprises the trigger assembly as described above. Herein, the trigger ring 1000 is in the form of a switch button of the atomizer, or the trigger ring 1000 is connected to the switch button of the atomizer, so that the trigger ring 1000 can move as the switch button is pressed. The delivery tube holder 2000 is in the form of a housing of the atomizer, or the delivery tube holder 2000 is connected to the housing of the atomizer, so that the delivery tube holder 2000 can rotate or spirally move as the housing rotates.

When the atomizer is in an initial position, the trigger assembly is located in the unlocked position, or when the atomizer is in a pre-trigger position, the trigger assembly is located in the locked position. Therefore, the delivery tube holder 2000 can rotate in the unlocked position, such that the atomizer moves from the initial position to the pre-trigger position, and the trigger ring 1000 can be pressed in the locked position to move, for example, moving substantially horizontally, such that the atomizer moves from the pre-trigger position back to the initial position.

In addition to the trigger assembly, the atomizer may further comprise other components, such as a housing 8000, a push switch 3000, a container 4000 configured to contain a liquid to be atomized, a spring 5000 for applying a thrust to the delivery tube holder 2000, a rotating member 6000 sleeved on the delivery tube holder 2000, a mouthpiece 7000 (which can be put in the user's mouth during use), a spraying assembly 9000 configured to perform atomization and spray an atomized liquid, and a dust cover 11000.

In the configuration shown in FIGS. 17 to 19, the atomizer may, but does not necessarily, have the following operation mode.

First, when the atomizer is in the initial position (in which case the trigger assembly is in the second unlocked position shown in FIGS. 15 and 16), the user holds the housing 8000 and rotates the housing by a certain angle, and the rotating member 6000 moves downwardly (for example, spirally) with the delivery tube holder 2000 as the housing 8000 rotates, until the trigger assembly moves to the locked position shown in FIGS. 9 to 11.

In this case, the atomizer is in the pre-trigger position, and the first locking surface 1110 abuts against the second locking surface 2110, so that the trigger ring 1000 and the delivery tube holder 2000 stop each other, and cannot further move without an external force. In the locked position, if the user does not deliberately apply an external force, the atomizer does not perform an atomization or spraying operation, even if the atomizer is subjected to an external force, such as an impact or shaking, or even accidents such as slipping from a high level. Therefore, the atomizer is effectively locked in this state.

When the user wants to use the atomizer, a radial inward thrust may be applied to the push switch 3000. Since the first locking surface 1110 and the second locking surface 2110 each are of a three-dimensionally inclined structure, the thrust is controlled within a rational range. Otherwise, in order to ensure a locking effect, the user has to apply a large operating force during unlocking, which undoubtedly leads to a decline in use experience and reduce user friendliness. In this case, the trigger ring 1000 moves as the push switch 3000 moves. It may be understood that since the first locking surface 1110 slides in three-dimensional directions on the second locking surface 2110, such a movement of the trigger ring 1000 with the push switch 3000 is not just a linear translation, but may be a curved translation with rotation. The curved translation allows for a relatively smooth switching of the trigger assembly between the locked position and the unlocked position. Preferably, during the sliding, the guide surface 1120 of the trigger ring 1000 is also in contact with the groove bottom of the locking notch 2100 and slides relatively. This dual contact sliding can reduce friction between the first locking surface 1110 and the second locking surface 2110, and thus prolong the service life of the trigger assembly.

Once the first locking surface 1110 of the trigger ring 1000 moves relative to the second locking surface 2110 to a position where they are no longer in contact with each other, the atomizer will enter a free position from the pre-trigger position (in which case the trigger assembly will be located in the first unlocked position shown in FIGS. 12 to 14). In the free position, the atomizer is not locked, and thus the delivery tube holder 2000 can move freely relative to the trigger ring 1000. However, in the atomizer, the delivery tube holder 2000 is constrained by the rotating member 6000 or the housing 8000 in the radial direction, and thus the delivery tube holder 2000 can only move relative to the trigger ring 1000 in the axial direction. Since the outer diameter of the delivery tube holder 2000 is less than the trigger ring 1000, the delivery tube holder 2000 can passively or actively pass through the trigger ring 1000, for example, by means of a spring force or a thrust to move vertically upwardly to a highest point, so that the trigger assembly, for example, returns to the second unlocked position shown in FIGS. 15 and 16, that is, the atomizer is restored to the initial position.

It should be understood that the internal structure and the components of the atomizer shown in the figures are exemplary rather than limiting structures, provided that a function of preventing misoperation or accidental triggering of the atomizer can be achieved herein. Therefore, the atomizer comprising the trigger assembly capable of achieving this function can have any structure and components, which are not limited to the components and arrangements in the above figures. In addition, the above operation mode of the atomizer is also exemplary, but not restrictive. When a component of the atomizer is omitted or replaced, the operation mode of the atomizer can be changed accordingly to meet use requirements.

The above are merely embodiments or examples of the present disclosure and thus do not limit the patent scope of the present disclosure, and any equivalent structural changes made under the concept of the present disclosure by utilizing the contents of the description and the accompanying drawings of the present disclosure, or direct/indirect applications in other related technical fields shall fall within the scope of protection of the present disclosure. Various elements in the embodiments or examples may be omitted or substituted by equivalent elements thereof. In addition, the steps may be performed in an order different from that described in the present disclosure. Further, various elements in the embodiments or examples may be combined in various ways. It is important that, as the technology evolves, many elements described herein may be replaced with equivalent elements that appear after the present disclosure.

## Claims

1. A trigger assembly for an atomizer, **characterized by** comprising a trigger ring (1000) and a delivery tube holder (2000),
wherein the trigger ring (1000) comprises a ring body (1200) and a first locking portion arranged on the ring body (1200), and at least one surface of the first locking portion forms a first locking surface (1110),
wherein the delivery tube holder (2000) comprises a tube holder body (2200) and a second locking portion arranged on the tube holder body (2200), and at least one surface of the second locking portion forms a second locking surface (2110),
wherein
the trigger assembly has a locked position and an unlocked position,
in the locked position, the first locking surface (1110) abuts against the second locking surface (2110), so that the trigger ring (1000) and the delivery tube holder (2000) stop each other, and
in the unlocked position, the first locking surface (1110) is not in contact with the second locking surface (2110), so that the delivery tube holder (2000) is movable in an axial direction relative to the trigger ring (1000).

2. The trigger assembly according to claim 1, **characterized in that**
the first locking portion is in the form of a locking protrusion (1100) extending out from at least one side of the trigger ring (1000), and
the second locking portion is in the form of a locking notch (2100) recessed on at least one side of the tube holder body (2200).

3. The trigger assembly according to claim 1, **characterized in that**
the first locking portion is in the form of a locking notch recessed on at least one side of the trigger ring (1000), and
the second locking portion is in the form of a locking protrusion extending out from at least one side of the tube holder body (2200).

4. The trigger assembly according to claim 1, **characterized in that** the trigger ring (1000) and the delivery tube holder (2000) stopping each other consists in that
the trigger ring (1000) prevents a vertical movement of the delivery tube holder (2000); and
the delivery tube holder (2000) prevents a horizontal movement of the trigger ring (1000).

5. The trigger assembly according to claim 1, **characterized in that** in the unlocked position, the delivery tube holder (2000) is movable through the trigger ring (1000) in the axial direction.

6. The trigger assembly according to claim 5, **characterized in that** an outer diameter of the delivery tube holder (2000) is less than or equal to an inner diameter of the trigger ring (1000).

7. The trigger assembly according to claim 1, **characterized in that** in the locked position, the trigger ring (1000) is arranged non-coaxially with respect to the delivery tube holder (2000), and in the unlocked position, the trigger ring (1000) is arranged coaxially with respect to the delivery tube holder (2000).

8. The trigger assembly according to claim 2, **characterized in that** the locking protrusion (1100) extends outwardly from a bottom side of the ring body (1200) of the trigger ring (1000), and the locking notch (2100) is arranged on a top side of the tube holder body (2200) of the delivery tube holder (2000).

9. The trigger assembly according to claim 2, **characterized in that** the first locking surface (1110) is in the form of a radially outward surface of the locking protrusion (1100), and the second locking surface (2110) is in the form of a radially inward surface of the delivery tube holder (2000).

10. The trigger assembly according to claim 2, **characterized in that** only one locking protrusion (1100) is provided on at least one side of the trigger ring (1000), and a plurality of locking notches (2100) are provided on at least one side of the delivery tube holder (2000).

11. The trigger assembly according to claim 10, **characterized in that** the plurality of locking notches (2100) are arranged at equal angular intervals in a circumferential direction on a side of the delivery tube holder (2000) that mates with the locking protrusion (1100).

12. The trigger assembly according to claim 2, **characterized in that** at least one of the first locking surface (1110) and the second locking surface (2110) is in the form of a flat inclined surface, and the inclined surface is inclined with respect to both a horizontal direction and a vertical direction.

13. The trigger assembly according to claim 12, **characterized in that** the first locking surface (1110) forms a first inclined locking surface, and the second locking surface (2110) forms a second inclined locking surface.

14. The trigger assembly according to claim 13, **characterized in that** the first inclined locking surface is in the form of a substantially trapezoidal inclined surface, and the second locking surface (2110) is in the form of a substantially parallelogram-shaped inclined surface.

15. The trigger assembly according to claim 12, **characterized in that** an included angle (β') between a normal direction (F') of the first locking surface (1110) and a first horizontal direction (X') of the trigger ring (1000) is in a range of 135° to 178°, preferably in a range of 155° to 175°; an included angle (γ') between the normal direction (F') of the first locking surface (1110) and a second horizontal direction (Y') of the trigger ring (1000) is in a range of 65° to 90°, preferably in a range of 70° to 85°; and an included angle (α') between the normal direction (F') of the first locking surface (1110) and a vertical direction (Z') of the trigger ring (1000) is in a range of 115° to 145°, preferably in a range of 120° to 140°.

16. The trigger assembly according to claim 12, **characterized in that** an included angle (β) between a normal direction (F) of the second locking surface (2110) and a first horizontal direction (X) of the delivery tube holder (2000) is in a range of 55° to 90°, preferably in a range of 67° to 87°; an included angle (γ) between the normal direction (F) of the second locking surface (2110) and a second horizontal direction (Y) of the delivery tube holder (2000) is in a range of 145° to 185°, preferably in a range of 155° to 178°; and an included angle (α) between the normal direction (F) of the second locking surface (2110) and a vertical direction (Z) of the delivery tube holder (2000) is in a range of 30° to 60°, preferably in a range of 40° to 55°.

17. The trigger assembly according to claim 12, **characterized in that** an included angle (A') between a projection of the first locking surface (1110) on a horizontal plane and a first radial direction (X') of the trigger ring (1000) is in a range of 5° to 50°, preferably in a range of 5° to 30°; and an included angle (B') between a projection of the first locking surface (1110) in a horizontal direction and a second radial direction (Y') of the trigger ring (1000) is in a range of 3° to 60°, preferably in a range of 5° to 20°.

18. The trigger assembly according to claim 17, **characterized in that** an included angle (C') between a projection of the first locking surface (1110) on a vertical plane and the axial direction (Z') of the trigger ring (1000) is in a range of 15° to 80°, preferably in a range of 30° to 50°.

19. The trigger assembly according to claim 12, **characterized in that** an included angle (A) between a projection of the second locking surface (2110) on a horizontal plane and a first radial direction (X) of the delivery tube holder (2000) is in a range of 5° to 60°, preferably in a range of 10° to 30°; and an included angle (B) between a projection of the second locking surface (2110) in a horizontal direction and a second radial direction (Y) of the delivery tube holder (2000) is in a range of 5° to 60°, preferably in a range of 5° to 25°.

20. The trigger assembly according to claim 18, **characterized in that** an included angle (C) between a projection of the second locking surface (2110) on a vertical plane and an axial direction (Z) of the delivery tube holder (2000) is in a range of 15° to 80°, preferably in a range of 35° to 55°.

21. The trigger assembly according to any one of claims 1 to 11, **characterized in that** at least one of the first locking surface (1110) and the second locking surface (2110) is in the form of a curved surface.

22. The trigger assembly according to claim 13, **characterized in that** the delivery tube holder (2000) further comprises an inclined guide surface (2120) on a side where the locking notch (2100) is provided, and the inclined guide surface (2120) has an edge shared with the second locking surface (2110).

23. The trigger assembly according to claim 22, **characterized in that** a radial dimension (d1) of the second locking surface (2110) is greater than a half of a radial wall thickness dimension (d2) of the delivery tube holder (2000), preferably greater than two thirds of the radial dimension (d2) of the delivery tube holder (2000).

24. The trigger assembly according to claim 23, **characterized in that** the inclined guide surface (2120) and the second locking surface (2110) jointly form a wall of the locking notch (2100).

25. The trigger assembly according to claim 22, **characterized in that** an included angle between a projection of the inclined guide surface (2120) in a horizontal direction and a second radial direction (Y) of the delivery tube holder (2000) is equal to an included angle (B) between a projection of the second locking surface (2110) in a horizontal direction and the second radial direction (Y) of the delivery tube holder (2000).

26. The trigger assembly according to claim 25, **characterized in that** an included angle (D) between a projection of the inclined guide surface (2120) on a horizontal plane and a first radial direction (X) of the delivery tube holder (2000) is in a range of 20° to 80°, preferably in a range of 40° to 65°.

27. An atomizer, **characterized by** comprising a trigger assembly according to any one of claims 1 to 26, for triggering the atomizer to spray an atomized liquid.

28. The atomizer according to claim 27, **characterized in that** the trigger ring (1000) is in the form of a switch button of the atomizer, or
the trigger ring (1000) is connected to the switch button of the atomizer, so that the trigger ring (1000) is movable as the switch button is pressed.

29. The atomizer according to claim 28, **characterized in that** the delivery tube holder (2000) is in the form of a housing of the atomizer, or
the delivery tube holder (2000) is connected to the housing of the atomizer, so that the delivery tube holder (2000) is rotatable as the housing rotates.

30. The atomizer according to claim 27, **characterized in that**
when the atomizer is in an initial position, the trigger assembly is located in the unlocked position; and
when the atomizer is in a pre-trigger position, the trigger assembly is located in the locked position.

31. The atomizer according to claim 30, **characterized in that**
the delivery tube holder (2000) is movable vertically in the unlocked position, such that the atomizer moves from the initial position to the pre-trigger position, and
the trigger ring (1000) is capable of being pressed in the locked position to move horizontally, such that the atomizer moves from the pre-trigger position back to the initial position.

32. The atomizer according to claim 30, **characterized in that** the atomizer further comprises a spring at a bottom of the delivery tube holder (2000), for applying a thrust to the delivery tube holder (2000) to urge the delivery tube holder (2000) to pass through the trigger ring (1000) in an axial direction.

33. The atomizer according to claim 27, **characterized by** further comprising:
a container configured to contain a liquid to be atomized, and
a spraying assembly configured to atomize the liquid sucked from the container and spray the atomized liquid.
